# EUROPEAN PATENT APPLICATION

(11) **EP 3 263 076 A1**
(43) Date of publication of application: **03.01.2018**
(21) Application number: 17174865.0
(22) Date of filing: 07.06.2017
(51) Int. Cl.: A61F 7/00

(54) **IMPLANTABLE THERMAL NEUROMODULATOR WITH WIRELESS POWERING AND WIRELESS COMMUNICATIONS AND FABRICATION METHOD THEREOF**

(30) Priority: 07.06.2016 PT 109436
(71) Applicant: Universidade do Minho, 4704-553 Braga (PT)
(72) Inventor: MENDES, Paulo Mateus, 4800-058 GUIMARÃES (PT); VALENTE GONÇALVES, Luís Miguel, 4800-058 GUIMARÃES (PT); DA COSTA DINIS, Hugo Daniel, 4800-058 GUIMARÃES (PT); PENTEADO NEIVA SILVA FERNANDES, José Miguel, 4800-058 GUIMARÃES (PT)
(74) Representative: Patentree, Lda.

(57) **Abstract**

Wireless brain-implantable device for brain thermal neuromodulation comprising a thermoelectric or thermomagnetic effect cooling device and a heat diffuser, wherein the cooling device comprises a heat source and a heat sink, wherein said heat source of the cooling device is thermally attached to the heat diffuser, wherein said heat diffuser is configured for diffusing heat to the brain and said heat sink is arranged for cooling a localized area of the brain. The cooling device may be a Peltier cooling device. The diffuser may be a wireless antenna for wireless communications. The diffuser may be a wireless antenna for wireless power transfer. It may comprise an electronic wireless power receiver connected to said antenna and connected for powering said cooling device. Said antenna may be microfabricated with self-folding technique. The cooling device may comprise a cooling spike for increasing brain cooling depth. A battery may be included for powering the device, in particular with a wireless electronic recharging circuit for recharging said battery.

## Description

### Technical field

The present disclosure relates to a wireless brain-implantable microdevice for achieving thermal neuromodulation of specific brain zones, in particular for reducing epilepsy seizures.

### Background

Advances in medicine and the knowledge of the human body took several research areas to a new level, where microdevices have been playing an important role. Implantable medical devices are becoming smaller, and new solutions on treatment, diagnostics, and even on the replacement of some normal functions, are getting an important biomedical field [1]. This market is currently growing 8% each year and it is contributing with some valuable new approaches that can revolutionize all medical treatments and diagnostics [2]. Today, this market asks for even smaller designs, more efficient circuits, and complex systems with sensors and actuators, all with wireless solutions. Such implantable devices could have hundreds of different usages, while smaller sizes could bring new applications to places that were before impossible to reach, thus leading to the monitoring or treatment of several conditions, including neurological disorders, cardiovascular problems, and ophthalmologic diseases [1].

Neuromodulation is the ability to manipulate the neurons' behavior, altering their biochemical processes or their electric signals. Such techniques are used to modify, normalize or modulate the electrical signals in order to accomplish different objectives, which are achieved by suppressing or improving the flow of action potentials. This is an expanding research area and three major applications are described in [1] as the three main purposes of neuromodulating devices: Prosthetics - Some devices can replace or improve the neural function. Therapeutics - the devices regulate neural activity for medical benefits. Neuroscience - Investigate the neural functions, observe their behavior and understand how they work. To change the electrical behavior of neurons, some different approaches are being studied for application in distinctive scenarios [1, 2]. Devices for Functional Electrical Stimulation (FES), where a current or a voltage is applied to a neuron, starting or changing is electrical activity, are the most common, and a well-known application is Deep Brain Stimulation [3].

Epilepsy is a disease where 25 to 30 percent of the patients continue to experience seizures after the best available therapy [3, 4], even after surgical treatment [5]. One method that was proposed with potential to treat medication RNPD cases is thermal neuromodulation [6]. Temperature control can change the cells' behavior and there are many different applications where increasing or decreasing the temperature can promote the modulation of the neurons' electrical signals. With this kind of neuromodulation, it is possible to, e.g., suppress epileptic seizures [3] or to test thermal stimulations on single neurons in vitro [7]. However, this method can also induce irreversible thermal damage on cells, so a complete control of the temperatures, localized areas and exposure times is imperative.

Epileptic seizure is a clinical manifestation of excessive and disorderly discharges of cortical neurons, neurons localized in the cerebral cortex, the outer layer of the cerebrum of the brain. Therefore, it is presumed that the triggers and signals that cause these seizures arise from regions outside of the cortex (thalamus and other subcortical regions). Consequently, the stimulation of the thalamus and other subcortical regions, located deeper within the brain, has been shown to control or even prevent seizures.

Document US 6923826 B2 discloses a neurosurgical device for thermal therapy using a thermoelectric cooler including a Peltier-effect cooler. However, the device is implanted in the skull and not configured to work wirelessly. Document US 20070225781 A1 and US 20030225442 A1 disclose and apparatus for cooling a region by 15°C and 30 °C, respectively, within the body using a Peltier junction. However, the heat is dissipated through an external heat exchanger (attached to a tubular body organ - vena cava).

Document US 2012290052 A1 discloses a skull-implanted device for brain cooling that uses thermally conductive prosthesis. However, the device produces a localized cooling of less than 2 °C or maybe less than 4 °C, the cooling location is limited by the need to have the heat pipe all the way between the desired cooling zone and the heating diffusion skull zone, being a large device and unpractical for the generated heat that requires diffusion and the need to cross the meninges and the cerebrospinal fluid.

These facts are disclosed in order to illustrate the technical problem addressed by the present disclosure.

### General Description

The present disclosure comprises a wireless implantable microdevice for achieving thermal neuromodulation.

The present disclosure comprises a cooling device comprising a hot plate and a cold plate for yielding temperature reductions, a heat sink for diffusing the heat generated, a signal receptor for wireless communications and wireless power transfer, and an integrated circuit, wherein said microcooler is integrated said signal receptor is integrated into said heat sink and said hot plate of said microcooler is attached to said heat sink.

It is disclosed a wireless brain-implantable device for brain thermal neuromodulation comprising a thermoelectric or thermomagnetic effect cooling device and a heat diffuser,
wherein the cooling device comprises a heat source and a heat sink,
wherein said heat source of the cooling device is thermally attached to the heat diffuser,
wherein said heat diffuser is configured for diffusing heat to the brain and said heat sink is arranged for cooling a localized area of the brain.

In an embodiment, the cooling device is a Peltier cooling device.

In an embodiment, said diffuser is a wireless antenna for wireless communications.

In an embodiment, said diffuser is a wireless antenna for wireless power transfer.

An embodiment comprises an electronic wireless power receiver connected to said antenna and connected for powering said cooling device.

In an embodiment, the wireless power receiver is a RF, radio-frequency, wireless power receiver.

In an embodiment, said antenna is microfabricated with self-folding technique.

In an embodiment, said heat diffuser is an aluminum plate, in particular with an area of 0.25 - 5 cm², in particular an area of 0.5 - 2 cm², further in particular 0.8 - 1.5 cm².

An embodiment comprises an integrated circuit for controlling said cooling device, wherein said integrated circuit is a complementary metal-oxide-semicondutor radiofrequency circuit.

In an embodiment, the heat sink of the cooling device comprises a cooling spike for increasing brain cooling depth.

In an embodiment, said spike has 800-1200 µm of length and 100-300 µm of diameter.

An embodiment comprises a plurality of said cooling devices.

An embodiment comprises a battery for powering the wireless implantable device, further in particular comprising a wireless electronic recharging circuit for recharging said battery.

In an embodiment, said cooling device is arranged to cool said heat sink at least 5°C, in particular at least 7°C, further in particular at least 9°C.

In an embodiment, said cooling device is arranged to cool said heat sink 5-12°C, in particular 5-9°C, further in particular sink 5-7°C.

The above described embodiments are combinable.

### Brief Description of the Drawings

The following figures provide preferred embodiments for illustrating the description and should not be seen as limiting the scope of invention.
**Figure 1****:** Perspective view of a schematic representation of an embodiment of the thermal modulator.
**Figure 2****:** Top view of a schematic representation of an embodiment of the thermal modulator.
**Figure 3****:** Schematic representation of an embodiment of one possible neuromodulation system architecture.
**Figure 4****:** Graph of temperature as a function of current obtained from simulation *versus* datasheet for MPC-D403 mode under relevant heat load conditions.
**Figure 5****:** Schematic representation of an embodiment of the model used to simulate the microcooler effect for this particular example, when the brain cells are considered as heat load.
**Figure 6****:** Temperature map on the brain cells with 50 mA in **(6a)** 5s **(6b)** 30s **(6c)** 100s obtained from simulation.
**Figure 7****:** Temperature map on the brain cells with 100 mA in **(7a)** 5s **(7b)** 30s **(7c)** 100s obtained from simulation.
**Figure 8****:** Temperature variation through A-A' cross-section, considering 50 mA and 100 mA currents.
**Figure 9****:** Temperature map on the brain cells obtained from simulation to test the effect of having: one microcooler in 5s **(a),** 30s **(b)** and 100s **(c);** two microcoolers in 5s **(d),** 30 **(e)** and 100s **(f).**
**Figure 10****:** Temperature map on the brain cells obtained from simulation when spikes are used on top of the microcooling device.
**Figure 11****:** Cooling volumes obtained from simulation for different microcoolers: one element, two elements, one element with spike, and two elements with spikes.

### Detailed Description

The present disclosure comprises a wireless implantable microdevice for achieving thermal neuromodulation.

Despite this device can push forward applications in different areas and, as an example it is proposed to open new horizons in thermal neuromodulation. The proposal consists on the use of a thermoelectric microcooler **2** (or any other cooling solution that may be integrated within the proposed device) based on Peltier effect as the cooling device, integrated with a RFCMOS chip **5** (or any other technology suitable for the fabrication of integrated circuits such as GaAs, SiC, CMOS, etc...) that hosts control, power, and communication electronics. The full system **1** also features an antenna **4** (or more than one antenna for different solutions).

As an example of the application of one wireless implantable microdevice **1** to achieve thermal neuromodulation, the use of this microdevice **1** on a rat brain is presented, keeping in mind that other applications that requires thermal modulation may use such device. In this particular example, the thermoelectric module **1** receives power from a radiofrequency (RF) signal to cool down the required site using the Peltier effect, but other mechanisms may be used to wirelessly power such device (for instance ultrasounds, low frequency signals, optical, magnetic, etc).

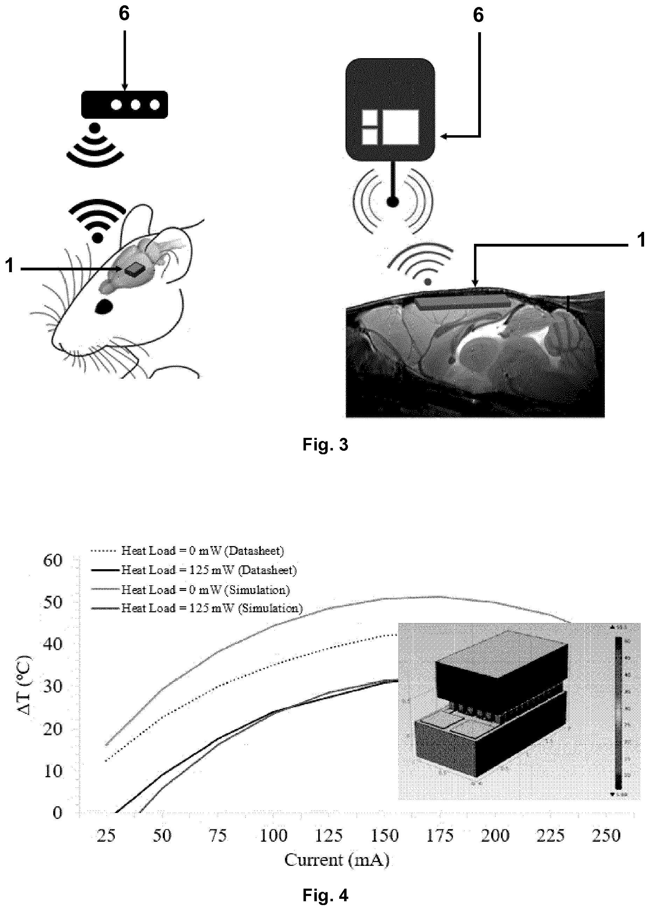

An example of a full system concept **1** (but other solutions may be used to obtain the full integration)for implanting a wireless temperature control system in a rat brain is shown in Figure 3.

The system is constituted by two modules: one external **6** and one internal comprising four components **1.** The internal module, our focus, is formed by three major parts: the signal receptor (antenna) **4,** the RFCMOS electronic circuit **5,** the microcooler actuator **2,** and a heat sink **3** wherein the antenna **4** is integrated and the microcooler **2** is attached to. The receptor **4,** or antenna, may be microfabricated with the self-folding technique, to obtain antenna size reduction, or any other antenna fabrication technique.

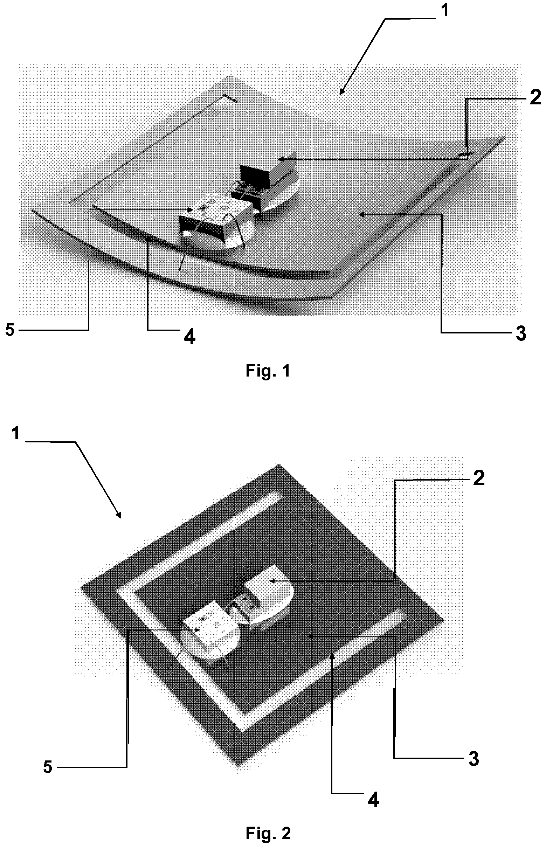

A goal of this example is to show a solution that can work at high frequencies with good efficiency, since this microsystem is expected to be powered wirelessly, and to work without, or with minimal use, of batteries (Figures 1 and 2).

In this example, the electronic circuit **5** converts the received AC signal to a DC signal that can be used to drive the microcooler **2,** but other solutions may be envisaged. With the use of amplifiers, filters and rectifiers, a DC signal can be extracted to power up the system, and activate the actuator **2.** This circuit **5** has to be efficient, without losses, and it has to work with low power signals. The core component of this circuit **5** is the thermal actuator, since it is responsible for thermal neuromodulation.

The temperature control is assured by a microcooler device **2.** This device's working principle in this embodiment is the thermoelectric effect (but may be any other equivalent effect), which states that when an electrical current is injected, the device will suffer temperature changes by heating up on one side of the device and cooling down on the opposite one. Nowadays, small microcooling devices that are able to operate with low currents and promote significant temperature gradients between their two sides are available. In this example, the selected microcooler device was the Micro MPC-D403. That device produces temperature differences up to 40 ºC between the hot and the cold side, by injecting less than 200 mA. This device was also chosen because it measures only 2 mm of length, being suitable to fit in a rat head, which was used for the first experiments. Since the implant is also being developed for rat studies, the system must to fit in a small volume (bellow 1 cm3), while being able to cool down one side to around 20 °C, and simultaneously guaranteeing the hot side does not heat the surrounding brain cells above 40 °C.

For the sake of miniaturization, the components **2-5** are integrated using microtechnologies such as multichip module technology and wire bonding, avoiding individual packaging of each element and promoting size reduction. However, any other microfabrication techniques may be considered for fabrication.

In this example, the thermal neuromodulator is built around a microcooler **2** (Micro MPC-D403), an in-house RFCMOS circuit **5,** and a metallic heat sink **3,** which is designed to integrate an antenna **4** for wireless communications and wireless power transfer. Considering a not yet optimized heat sink **3,** the full system volume **1** was designed to be smaller than 10 x 10 x 3 mm³.

To evaluate the neuromodulator's thermal performance, brain modelling was required to allow the full thermal problem analysis. The three main variables considered for brain heat transfer simulations are shown in Table 1.

**Table 1, hereinafter, shows the values for heat capacity - Cp, density - ρ and thermal conductivity-λ used in to simulate the materials of the neuromodulator 2.**

| **Variable** | **Value** |
|---|---|
| ***Cp* (J/K)** | 3700 |
| ***ρ* (kg/m³)** | 1050 |
| ***λ* (W/(m.K))** | 0.527 |

Also, due to the brain's thermal regulation mechanism, the convective heat generated by blood perfusion was considered to be equal to 0.029 W/cm³/K, and the metabolic heat equal to 0.025 W/cm³ . Those values represent the temperature control due to the brain blood vessels, responsible to maintain 37°C on the brain cells at all times. Since brain cells are known to be at a temperature of 37°C, the model starts with that temperature.

The temperature controller **6,** and core of the proposed microsystem **2,** is a commercially available microcooler (Micro MPC-D403), which is based on the thermoelectric effect. It will be the element in contact with the neural cells, and must be able to drive the cold side down to 20 °C (from 37 °C). The computational model was designed with the same dimensions as the microcooler device **2** and with the materials commonly used in thermoelectric applications. So, the plates of the microcooler device **2** were constituted by alumina (electric isolator and a good thermal conductor), while the pads and the thermoelectric material were made of copper and bismuth telluride (Bi₂Te₃), respectively. With this computational model for thermoelectric simulations, it is possible to simulate interactions between the microcooler **2** and, for example, brain cells. Applying the thermoelectric equations, including the *Seebeck,* the *Peltier* and the *Joule* effects, an accurate thermoelectric simulation may be achieved only if the correct material properties are known.

**Table 2, hereinafter, shows values of material properties used in the present simulation.**

| **Material** | **Variables** | | | | |
|---|---|---|---|---|---|
| | ***Cp (J*/*****K**)* | ***ρ (kg*/*m³)*** | ***λ (W*/*(m.K))*** | ***S (V*/*K)*** | ***σ (S*/*m)*** |
| Alumina | 900 | 3900 | 27 | N.A. | N.A. |
| Copper | 385 | 8700 | 400 | 6.5e-6 | 5.99e7 |
| Bi₂Te₃ | 154 | 7700 | 1.75 | ±170e-6 | 0.99e5 |
| Brain Tissue | 3700 | 1050 | 0.527 | N.A. | N.A. |

*Cp* is the heat capacity, *ρ* is the density of the material, *λ* is the thermal conductivity, *S* is the *Seebeck* coefficient, and *σ* is the electrical conductivity. Brain characteristics were obtained from [8, 9], while other materials were standard values, adjusted to the microcooler device **2** when necessary. The microcooler device **2** was simulated with the aforementioned thermoelectric equations in order to provide approximate results to those presented in the datasheet. In Figure 4, it is possible to compare the achieved temperature difference between the microcooler plates, both for the data presented on the datasheet and the results obtained in simulation.

In figure 4, the curves represent the temperature difference (between the hot side and the cold side) presented in the device's datasheet, for a hot side temperature of 40°C, and the simulated results, in equivalent conditions. The dashed lines represent the microcooler **2** with a heat load of 0 mW in the cold side, and the solid line represents the same conditions but with a heat load of 125 mW on the cold side. The brain heat load value can fit between 0 mW and 125 mW, therefore it is expected that the behavior of the simulated microcooler **2** will be approximately equivalent to the real application scenario. It's also possible to observe that, for a temperature difference of approximately 20 °C, the microcooler requires a current of approximately 50 mA.

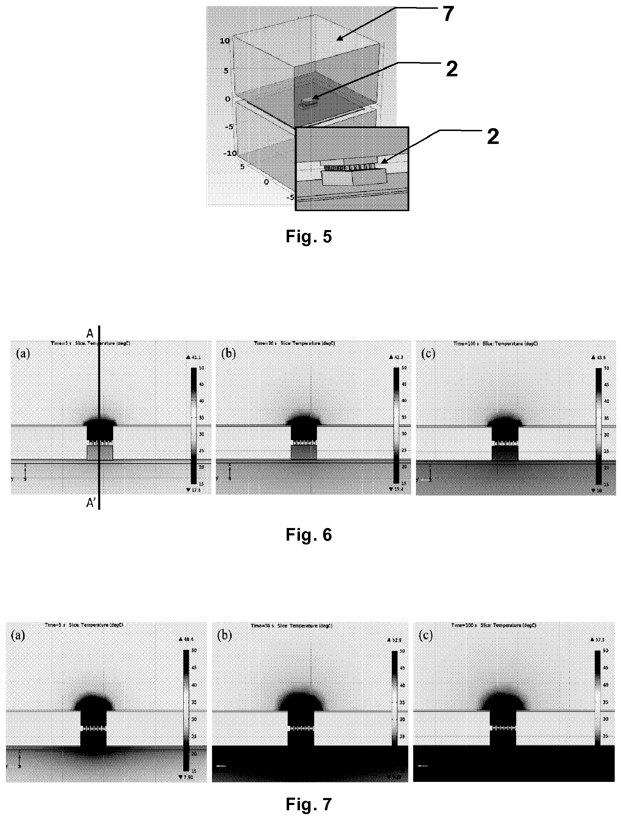

After validation of the microcooler element **2,** the brain cells **7** were added, as shown in Figure 5, in this particular example being modeled as blocks of 12 mm x 10 mm, while the microcooler device **2** was placed in between them **7.** It was considered that the microcooler device is insulated in the lateral faces. This means that the sides of the microcooler device do not lose heat, and only the surfaces were exposed to the brain cells **7.** In the brain heat transfer simulations, in order to make it more realistic, the thermal regulation caused by blood flow was considered using a convenient heat flow. Convective heat generated by blood perfusion was considered to be 0.029 W/cm³/K and the metabolic heat was set as 0.025 W/cm³ [10].

To avoid overheating of nearby neurons on the hot side, the microcooler **2** was attached to a heat sink to diffuse the heat generated over a larger area. Consequently, an aluminum plate **3** with 1 cm² was added to make the heat extraction. The aluminum heat sink **3** was added to the microcooler hot side and the full device was placed between cells of the brain **7.** Since it is well known that a microcooler **2** has an optimal operating point, the model was then used to inject different currents, giving us a perspective of the brain cells' expected temperature gradients. After analyzing Figure 4 (and knowing that the brain has a heat load between 0 mW and 125 mW), to cool it down to 20 °C, a current between 50 mA and 100 mA was expected, so these two currents were simulated for 100s. It is also known that the brain cells cannot reach temperatures higher than 43°C and bellow 0°C, as to avoid irreversible thermal damage. The device is preferably made or coated with a biocompatible material, e.g. platina.

Four microcooling solutions were assessed: single or double microcooler, and with and without spikes. Many other solutions may be developed around the proposed microdevice **2.** The analysis was evaluated at three different instants, while using a current of 50 mA to drive the microcooler **2.** This current value was used since, both from Micro MPC-D403 datasheet and Figure 4, a temperature differential of approximately 20 ºC is achieved when the device is powered by approximately 50 mA.

The obtained values when injecting 50 mA and 100 mA, respectively, during 5, 30, and 100 s, are represented in Figure 6 and 7. Observing the first results shown in Figure 6, it is possible to see that the hot side temperature has more effect in the cells than the cold side in a short time. Letting the microcooler **2** turned on for a longer time, the hot side temperature increases, but the cooled area only slightly expands. Comparing the 30 s with the 100 s exposure instants, we can assume that the cold side differences between them are insignificant. In all cases the temperature did not exceed 43 °C and did not drop below 0 °C, meaning that these experiments will not inflict thermal damage on the brain cells. Observing the second case (injection of 100 mA in the microcooler - Figure 7), we can immediately notice that larger cooled areas were achieved with even lower temperatures when compared with the results with 50 mA, but the hot side got hotter, which is easily explained by the joule effect due to more current being injected in the system. This led to a temperature raise on the hot side, where the obtained values were above 43 °C and, consequently, the brain cells could suffer irreversible damage and even be destroyed, if exposure time is not controlled.

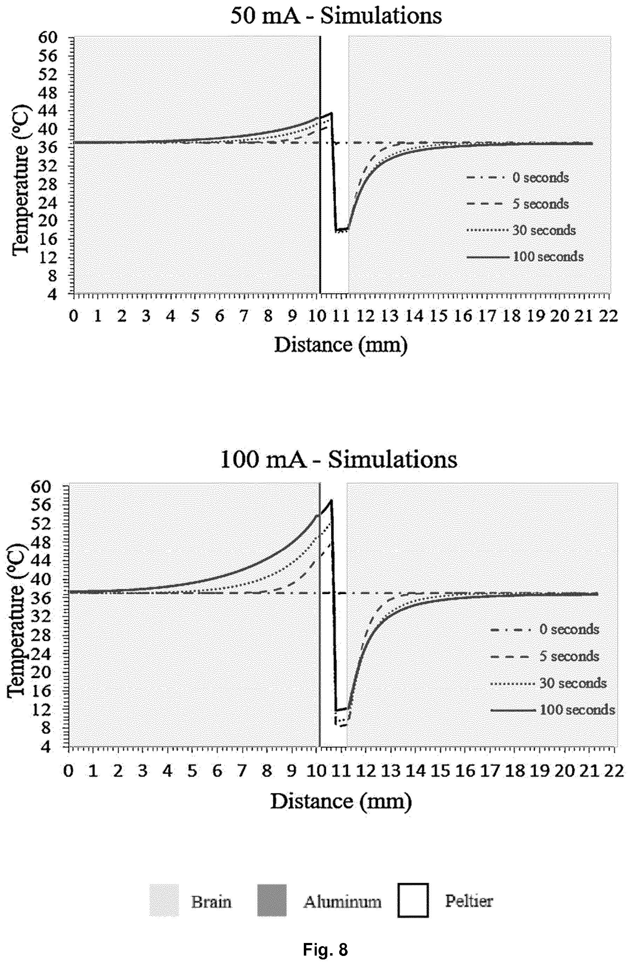

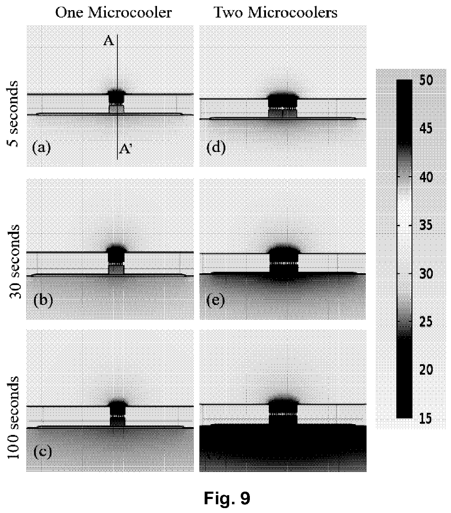

In order to easily find the precise values of temperature that were achieved and the depths at which they were felt, a cross-section was selected right through the center of the microcooler device **2** (defined by the cross section A-A' in Figure 6 (a)). In Figure 8 the temperature results are shown, and we can now clearly see that with a 50 mA current the brain never reached 43 °C. On the cold side it was possible to push areas of the brain down to 20ºC and to cool 1 mm of brain tissue bellow 30°C. It is also possible to say that the differences between 30 and 100 s are negligible and that increasing the exposure time has more effect on the hot side of the system. Looking now to the 100 mA plot, as expected, the hot side temperatures exceed 43°C even after 5 s exposure time, leading to conclude that higher currents are more difficult to control for these applications.

The temperature evolution with time is important because it impacts how long the device needs to operate in order to stop seizures, therefore influencing how much energy is required to stop the seizure. This translates directly on battery dimensions and/or device lifetime between recharges. Also, exposing brain cells to high temperatures for long time periods may lead to cell damage. Figure 9 shows the results when one or two microcoolers were used, at time stamps 5, 30 and 100 seconds.

Observing the 100 s instant, it can be observed that the volume of cooled cells is not significantly bigger than the volume at 30 s, but the hot side shows higher values of temperature, which may be harmful to cells. So, this exposure time has increased damage risks without any benefits to the experiment. So, turning on the microcooler **2** for 30 s is enough guarantee the required temperature in the cold side, without exceeding the hot side temperature limits.

In focal cooling, achieving greater depths can be interesting and more important than getting bigger areas, because seizures can begin in nerve cells that are some millimeters bellow the grey matter of the brain [4]. Figure 8 shows the temperature variations in the cross section A to A' (shown on Figure 6). The cooled area may double when using two microcoolers, but the depth of brain cells that are cooled is not significantly larger, as is shown in Figure 9.

Since the neuromodulation obtained will depend on the depth of the cooled region, it is important to provide solutions to control the volume affected by temperature changes. To overcome the problem of low cooling depths, it was proposed to add some spikes that can get inside the grey matter, and more easily reduce cells temperature. So, in each microcooler device **2,** one small spike with 1000 µm of length and 200 µm of diameter was added to assess if the cooled area and depth can be increased. This small spike was simulated as aluminum (but other materials may be used for the sake of biocompatibility) because this material is a good thermal conductor.

Looking at the results for two devices, at 30 s, and regarding achieved temperatures versus distance from the microcooler **2,** similar results can be observed as those for one device. However, the required power doubles, since each device requires 50 mA to operate. As it does not cool down two times faster, this means more energy required to achieve the same temperature, therefore larger batteries are necessary and larger device volume would come as a consequence.

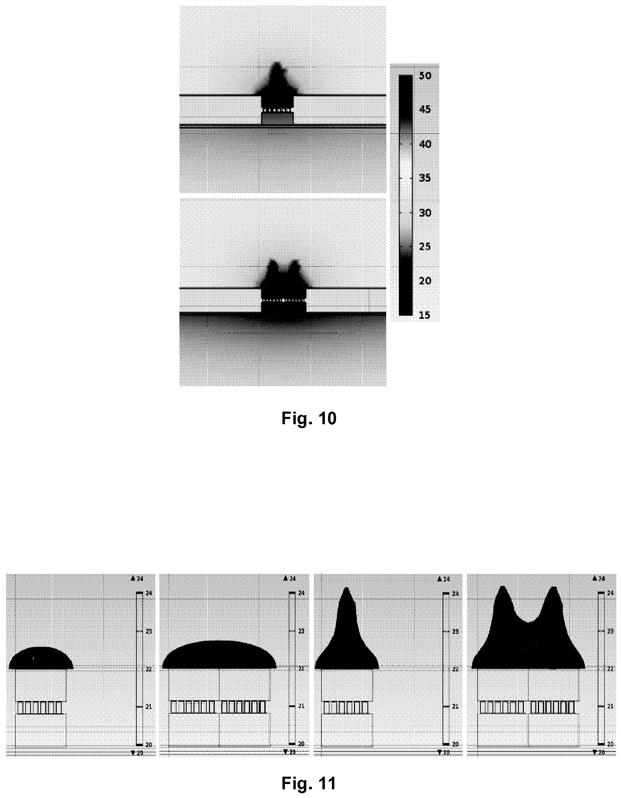

Observing the obtained results, it is obvious that the spikes increased the depth of cooled tissue, both with one and two microcoolers, while the hot side was mostly unaltered. To check and compare the areas and volumes of cooled tissue, Figure 11 may be used.

In Figure 11, it is possible to make a final comparison on the volume of tissue that is indeed cooled bellow 25 °C for each simulation that was made in this work. As it can be seen, the introduction of spikes produces a big effect, even considering that part of the volume that is occupied by the spikes themselves. These results show that the cooling depth can be increased resorting on the use of spikes on the top of the microcoolers **2.**

Power is one of the most important parameters when building a wireless microsystem, and the microcooler device **2** will probably be the one that requires more energy to work properly. From simulations, it was also possible to obtain the electrical potential values between the microcooler feeding pads, and the results are presented in Table 3.

**Table 3, hereinafter, shows the values of voltage on the microcooler device 2 obtained from the simulations.**

| **Current** | **5s** | **30s** | **100s** |
|---|---|---|---|
| 50 mA | 1.22 V | 1.24 V | 1.25 V |
| 100 mA | 2.36 V | 2.43 V | 2.49 V |

With the values presented in the previous table, it is possible to estimate that for 50 mA, the power consumed by this microcooler device is between 61 mW and 62.5 mW. For 100 mA, the power consumption is between 236 mW and 249 mW. These values are higher than usually found on wireless implantable devices, but it does not mean that this device cannotbe used with that same objective. As described in [11], in a small implantable device the power delivered to the device cannotgo above a few mW respecting the specific absorption rate (SAR). To overcome this problem, a small battery will be required to provide energy for discharges, which may be wirelessly recharged between seizures.

This document proposes the use of microfabrication technologies to obtain a microsystem for thermal neuromodulation **1.** The presented solution core device size, a microcooler **2,** is in the mm scale, where the expected full device volume **1** is expected to be bellow 10 x 10 x 3 mm³. After testing, it was possible to conclude that the implemented system is able to cool one millimeter of brain tissue **7** bellow 30 °C, with a current of 50 mA, dissipating only 62.5 mW, while keeping the hot side below safe values. In fact, it was possible to get temperatures near 20°C in some brain cells, keeping the hot side below 43°C. According to [3], this means that the proposed system has the potential to suppress epilepsy seizures without using big external heatsinks, as is common in other works [3].

Since different brain tissue thicknesses are under consideration, solutions to control the cooling depth that may achieved by the microcooler **2,** were also investigated. Solutions with multiple coolers were analysed, as well as the use of spikes, instead of flat coolers. Equally important was the analysis of the time that the microcooler **2** must be kept switched on. It was essential to understand how to avoid exceeding the temperature threshold in the hot side, how long it may take to stop seizures, and to provide guidelines about the required rechargeable battery size. The power consumption results lead us to conclude that a small battery will be enough to store the required energy between each discharge. Considering that epilepsy seizures are a not a very frequent event, the battery could be wirelessly recharged with the necessary energy between each seizure event.

It may be concluded that the proposed device is suitable for use in neuromodulation studies, or other thermal modulations applications that such device may be used for, and, as an example, a detailed analysis was presented for an application on a rat brain.

The disclosure should not be seen in any way restricted to the embodiments described and a person with ordinary skill in the art will foresee many possibilities to modifications thereof. The above described embodiments are combinable. The following claims further set out particular embodiments of the disclosure.

### References

[1] S. Luan, I. Williams, K. Nikolic, T. G. Constandinou, "Neuromodulation: present and emerging methods" in frontiers in Neuroengineering, Vol. 7, July 2014.
[2] M. Gofeld, "New Horizons in Neuromodulation" in Current Pain and Headache Reports, 18:397, March 2014.
[3] D. E. Hardesty, H.A. Sackeim "Deep brain stimulation in movement and psychiatric disorders". Biol. Psychiatry 61, p831-835, 2007.
[4] G. Bonmassar, et al, "Microscopic magnetic stimulation of neural tissue", Nature Communications 3, 921, June 2012.
[5] Y. Tufail, et al, "Transcranial Pulsed Ultrasound Stimulates Intact Brain Circuits", Neuron 66, p681-694, June 2010.
[6] M. Fujii, H. Fujioka, et al, "Application of Focal Cerebral Cooling for the Treatment of Intractable Epilepsy", Neurol Med Chir (Tokyo) 50, p839-844, 2010.
[7] F. Reverter, T. Prodromakis, et al, "Design Considerations for a CMOS Lab-on-Chip Microheater Array to Facilitate the in vitro Thermal Stimulation of Neurons", in Circuits and Systems (ISCAS), 2014 IEEE International Symposium on , vol., no., pp.630-633, 1-5 June 2014
[8] L. Zhu and A. Rosengart, "Cooling penetration into normal and injured brain via intraparenchymal brain cooling probe: theoretical analysis," Heat Transfer Engineering, 29, 284-294 (2008).
[9] Mraovitch, S., C. ladecola, and D. J. Reis. "Vasoconstriction unassociated with metabolism in cerebral cortex elicited by electrical stimulation of the parabrachial nucleus in rat.", J. Cerebral Blood Flow and Metab., 3 (Suppl. 1), S196-S197, 1983.
[10] I. Osorio, F. Chang, N. Gopalsami, "Seizure control with thermal energy? Modeling of heat diffusivity in brain tissue and computer-based design of a prototype mini-cooler" Epilepsy Behav, pp203-211, Sep 2009.

## Claims

1. Wireless brain-implantable device for brain thermal neuromodulation comprising a thermoelectric or thermomagnetic effect cooling device and a heat diffuser,
wherein the cooling device comprises a heat source and a heat sink,
wherein said heat source of the cooling device is thermally attached to the heat diffuser,
wherein said heat diffuser is configured for diffusing heat to the brain and said heat sink is arranged for cooling a localized area of the brain.

2. Wireless implantable device according to the previous claim, wherein the cooling device is a Peltier cooling device.

3. Wireless implantable device according to any of the previous claims, wherein said diffuser is a wireless antenna for wireless communications.

4. Wireless implantable device according to any of the previous claims, wherein said diffuser is a wireless antenna for wireless power transfer.

5. Wireless implantable device according to the previous claim, further comprising an electronic wireless power receiver connected to said antenna and connected for powering said cooling device.

6. Wireless implantable device according to the previous claim, wherein the wireless power receiver is a RF, radio-frequency, wireless power receiver.

7. Wireless implantable device according to any of the claims 3-7, wherein said antenna is microfabricated with self-folding technique.

8. Wireless implantable device according to any of the previous claims, wherein said heat diffuser is an aluminum plate, in particular with an area of 0.25 - 5 cm², in particular an area of 0.5 - 2 cm², further in particular 0.8 - 1.5 cm².

9. Wireless implantable device according to any of the previous claims, further comprising an integrated circuit for controlling said cooling device, wherein said integrated circuit is a complementary metal-oxide-semicondutor radiofrequency circuit.

10. Wireless implantable device according to any of the previous claims, wherein the heat sink of the cooling device comprises a cooling spike for increasing brain cooling depth.

11. Wireless implantable device according to the previous claim, wherein said spike has 800-1200 µm of length and 100-300 µm of diameter.

12. Wireless implantable device according to any of the previous claims, comprising a plurality of said cooling devices, in particular each cooling device comprising a cooling spike for increasing brain cooling depth.

13. Wireless implantable device according to any of the previous claims, comprising a battery for powering the wireless implantable device, further in particular comprising a wireless electronic recharging circuit for recharging said battery.

14. Wireless implantable device according to any of the previous claims, wherein said cooling device is arranged to cool said heat sink at least 5°C, in particular at least 7°C, further in particular at least 9°C.

15. Wireless implantable device according to any of the previous claims, wherein said cooling device is arranged to cool said heat sink 5-12°C, in particular 5-9°C, further in particular sink 5-7°C.
